# EUROPEAN PATENT SPECIFICATION

(11) **EP 1 673 237 B1**
(45) Date of publication and mention of the grant of the patent: **15.08.2012**
(21) Application number: 03752357.8
(22) Date of filing: 12.09.2003
(51) Int. Cl.: B43K 5/02, B43K 5/14, B43K 5/00, A46B 11/06, A46B 11/00, B05C 21/00, A61M 35/00, B08B 1/00, A45D 34/04, A45D 40/24, A61F 13/38, A61M 37/00, B43K 8/03, B43M 11/06

(54) **APPLICATOR WITH TWO LIQUIDS**
APPLIKATOR MIT ZWEI FLÜSSIGKEITEN
APPLICATEUR A DEUX LIQUIDES

(43) Date of publication of application: 28.06.2006
(73) Proprietor: Unidose Systems, Inc., Rancho Cucamonga, CA 91730 (US)
(72) Inventor: Tsaur, Garry, Rowland Heights, CA 91748 (US)
(74) Representative: Viering, Jentschura & Partner
(86) International application number: PCT/US2003/028846
(87) International publication number: WO 2005/035267

(56) References cited:
- EP-A1- 0 553 534
- EP-A1- 1 405 579
- US-A- 3 519 364
- US-A- 3 724 018
- US-A- 4 740 194
- US-A- 5 490 736
- US-A- 5 702 035
- US-B1- 6 729 786

## Description

### BACKGROUND-FIELD OF INVENTION

The present invention relates generally to a swab applicator. More specifically the present invention relates to a hollow tube swab applicator for storing and applying two liquids.

### BACKGROUND-DESCRIPTION OF RELATED ART

Swab applicator generally comprises of a tubular handle with a formed absorbent tip at one or both ends of the tubular handle. The absorbent tip may be made of cotton or a foam absorbent material. The tubular handle may be made of wood, paper, or plastic and it may be solid or hollow.

Swab applicators have a variety of applications. Swab applicators are a convenient and sanitary means for applying a variety of substances such as liquids, lotions, creams, and various chemicals and medications. Generally the applicator tip of a dry swab applicator is first placed in contact with the liquid to be applied for the applicator tip to absorb the liquid.

Subsequently, the moisturized applicator tip is placed in contact with the surface to apply the absorbed liquid to the surface. US Patent No. 5,702,035 issued to Tsao shows one design wherein the hollow tubular handle encloses a liquid that can be subsequently released into the applicator tip for application. However, the swab applicator with a hollow tubular handle disclosed in US Patent No. 5,702,035 only contains one liquid. Some applications require two separate liquids to be stored separately and mixed just prior to application or applied in sequence.

Document EP 1 405 579 A1 which is a prior art document under Article 54(3) EPC discloses an applicator including at least one tube and two different substances which are separated from each other by an extra component such as a liquid or a powder which is expelled out of the tube together with the substances during use. Document EP 0 553 534 A1 discloses a tubular container having multiple permeable absorbents which are separated from each other by a separating element such as a permeable tape which wraps an absorbent. This separating element has to be removed to enable a liquid to flow out from the tubule. Document US 4,740,194 discloses a self-contained liquid swab having two liquids that are separated from each other by an air-gap. In order to release the liquids, it is necessary to squeeze the mid-section of the tube.

### SUMMARY OF THE INVENTION

The present invention is an applicator with two liquids comprising a hollow elongated tubular handle with a sealed end and an open end with an applicator tip affixed to one or both ends of the hollow elongated tubular handle. An opening means is provided near the sealed end of the hollow elongated tubular handle. A first liquid is enclosed inside the hollow elongated tubular handle separated from the open end by a viscous substance and a second liquid is absorbed in the applicator tip. When air is allowed to enter near the sealed end through the opening means and where the applicator tip is only partially saturated with the second liquid, the first liquid in the hollow elongated tubular handle will flow through the viscous substance and commingle with the second liquid in the applicator tip to be applied. If the applicator tip is saturated with the second liquid, the first liquid will not immediately flow into the applicator tip but, instead, the second liquid will be first partially depleted through application before the first liquid will flow into the applicator tip for application.

The invention provides an applicator with two liquids according to claim 1. Further embodiments of the invention are described in the dependent claims.

### BRIEF DESCRIPTION OF THE DRAWINGS

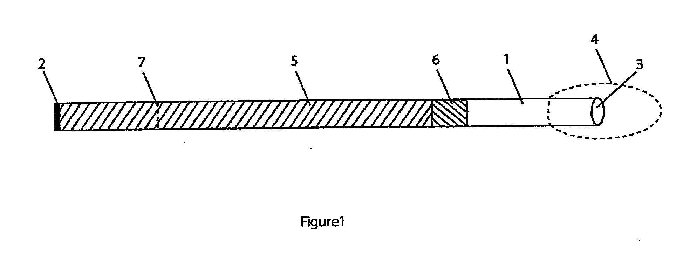

Figure 1 shows the preferred embodiment of the applicator with two liquids.

### DETAILED DESCRIPTION OF THE PREFERRED EMBODIMENT

Figure 1 shows the preferred embodiment of the present invention. In the preferred embodiment, the applicator with two liquids comprises of a hollow elongated tubular handle **1** with a sealed end **2** and an open end **3.** An applicator tip **4** is affixed to the open end **3** of the hollow elongated tubular handle **1.** A first liquid **5** is disposed within the hollow elongated tubular handle **1** separated from the open end **3** by a viscous substance **6.** The preferred viscous substance **6** is silicone but other suitable viscous substance may also be utilized. An opening means **7** such as a score line is positioned near the sealed end **2.**

A second liquid is absorbed into the applicator tip **4.** If the second liquid is nonevaporative, such as oil-based liquid, the applicator tip **4** will not require further packaging to prevent evaporation of the second liquid. If the second liquid is an evaporative liquid, such as water-based liquid or alcohol, additional packaging may be necessary to prevent evaporation of the second liquid from the applicator tip **4.**

Furthermore, if the applicator tip **4** is fully saturated with the second liquid the applicator tip **4** may act as a stopper and prevent the exit of the first liquid **5** at the first instance of opening. In this instance, the second liquid will have to be applied first. Upon partial depletion of the second liquid, the first liquid **5** will then begin to flow into the applicator tip for application next. This embodiment is particularly suited for applications where one liquid, such as rust remover or cleaner, must be applied first before a second liquid, such as a lubricant or a protectant is to be applied. The applicator tip **4** may also be partially moistened with the second liquid to allow the first liquid **5** to flow and exit the hollow elongated tubular handle **1** and to commingle with the second liquid to be applied at the same time. This embodiment is particularly suitable for applications where two separate liquids must be mixed and used together.

Upon opening of the hollow elongated tubular handle **1** at the opening means **7** the first liquid 5 will flow through the viscous substance **6,** which will spread open and adhere to the inside of the hollow elongated tubular handle **1** and remain essentially at its original position, and into the absorbent tip **4** to commingle with the second fluid for application.

Although the description above contains many specificities, these should not be construed as limiting the scope of the invention but as merely providing illustrations of some of the presently preferred embodiments of this invention. Thus the scope of the invention should be determined by the appended claims and their legal equivalents, rather than by the examples given.

## Claims

1. An applicator with two liquids comprising a hollow elongated tubular handle (1) with a sealed end (2) and an open end (3) with an opening means (7) positioned near the sealed end (2) and an applicator tip (4) affixed to said open end (3) of the hollow elongated tubular handle (1) with a first liquid (5) disposed within the hollow elongated tubular handle (1) and a second liquid absorbed into said applicator tip (4) , **characterised in that** the first liquid (5) is separated from the open end (3) by a viscous substance (6) wherein upon opening of the hollow elongated tubular handle (1) through the opening means (7), the first liquid (5) will flow through the viscous substance (6) and into the said absorbent tip (4) for application with the second liquid the viscous substance remaining essentially at its original position.

2. An applicator with two liquids as in claim 1, wherein a second applicator tip is affixed to said sealed end (2) of the hollow elongated tubular handle (1).

3. An applicator with two liquids as in claim 1, wherein the applicator tip (4) is made of an absorbent material.

4. An applicator with two liquids as in claim 1, wherein said opening means (7) is a score line.

5. An applicator with two liquids as in claim 3, wherein said opening means (7) is a score line.

6. An applicator with two liquids as in claim 1, wherein said viscous substance (6) is silicone.

7. An applicator with two liquids as in claim 3, wherein said viscous substance (6) is silicone.

8. An applicator with two liquids as in claim 4, wherein said viscous substance (6) is silicone.

9. An applicator with two liquids as in claim 1, wherein said applicator tip (4) is saturated with said second liquid.

10. An applicator with two liquids as in claim 1, wherein said applicator tip (4) is only partially saturated with said second liquid.

11. An applicator with two liquids as in claim 1, wherein said opening means (7) is a frangible section and said viscous substance (6) is silicone.

12. An applicator with two liquids as in claim 11, wherein a second applicator tip is affixed to said sealed end (2) of the hollow elongated tubular handle (1).

13. An applicator with two liquids as in claim 11, wherein said applicator tip (4) is saturated with said second liquid.

14. An applicator with two liquids as in claim 11, wherein said applicator tip (4) is only partially saturated with said second liquid.

## Patentansprüche

1. Ein Applikator mit zwei Flüssigkeiten, aufweisend einen hohlen langgestreckten rohrförmigen Griff (1) mit einem abgedichteten Ende (2) und einem offenen Ende (3), mit einem Öffnungsmittel (7), das in der Nähe des abgedichteten Endes (2) positioniert ist, und einer Applikatorspitze (4), die an dem offenen Ende (3) des hohlen langgestreckten rohrförmigen Griffs (1) befestigt ist, mit einer ersten Flüssigkeit (5), die innerhalb des hohlen langgestreckten rohrförmigen Griffs (1) angeordnet ist, und einer zweiten Flüssigkeit, die in die Applikatorspitze (4) absorbiert ist, **dadurch gekennzeichnet, dass** die erste Flüssigkeit (5) durch eine viskose Substanz (6) von dem offenen Ende (3) getrennt ist, wobei beim Öffnen des hohlen langgestreckten rohrförmigen Griffs (1) durch das Öffnungsmittel (7) die erste Flüssigkeit (5) durch die viskose Substanz (6) hindurch und in die Absorptionsspitze (4) hineinströmt, zum Applizieren mit der zweiten Flüssigkeit, wobei die viskose Substanz im Wesentlichen in ihrer ursprünglichen Position bleibt.

2. Ein Applikator mit zwei Flüssigkeiten gemäß Anspruch 1, wobei eine zweite Applikatorspitze an dem abgedichteten Ende (2) des hohlen langgestreckten rohrförmigen Griffs (1) befestigt ist.

3. Ein Applikator mit zwei Flüssigkeiten gemäß Anspruch 1, wobei die Applikatorspitze (4) aus einem Absorptionsmaterial hergestellt ist.

4. Ein Applikator mit zwei Flüssigkeiten gemäß Anspruch 1, wobei das Öffnungsmittel (7) eine Kerblinie ist.

5. Ein Applikator mit zwei Flüssigkeiten gemäß Anspruch 3, wobei das Öffnungsmittel (7) eine Kerblinie ist.

6. Ein Applikator mit zwei Flüssigkeiten gemäß Anspruch 1, wobei die viskose Substanz (6) Silikon ist.

7. Ein Applikator mit zwei Flüssigkeiten gemäß Anspruch 3, wobei die viskose Substanz (6) Silikon ist.

8. Ein Applikator mit zwei Flüssigkeiten gemäß Anspruch 4, wobei die viskose Substanz (6) Silikon ist.

9. Ein Applikator mit zwei Flüssigkeiten gemäß Anspruch 1, wobei die Applikatorspitze (4) mit der zweiten Flüssigkeit durchtränkt ist.

10. Ein Applikator mit zwei Flüssigkeiten gemäß Anspruch 1, wobei die Applikatorspitze (4) nur teilweise mit der zweiten Flüssigkeit durchtränkt ist.

11. Ein Applikator mit zwei Flüssigkeiten gemäß Anspruch 1, wobei das Öffnungsmittel (7) ein zerbrechlicher Abschnitt ist und die viskose Substanz (6) Silikon ist.

12. Ein Applikator mit zwei Flüssigkeiten gemäß Anspruch 11, wobei eine zweite Applikatorspitze an dem abgedichteten Ende (2) des hohlen langgestreckten rohrförmigen Griffs (1) befestigt ist.

13. Ein Applikator mit zwei Flüssigkeiten gemäß Anspruch 11, wobei die Applikatorspitze (4) mit der zweiten Flüssigkeit durchtränkt ist.

14. Ein Applikator mit zwei Flüssigkeiten gemäß Anspruch 11, wobei die Applikatorspitze (4) nur teilweise mit der zweiten Flüssigkeit durchtränkt ist.

## Revendications

1. Applicateur à deux liquides comprenant un manche tubulaire allongé creux (1) avec une extrémité scellée (2) et une extrémité ouverte (3) avec un moyen d'ouverture (7) positionné près de l'extrémité scellée (2) et un embout applicateur (4) fixé à ladite extrémité ouverte (3) du manche tubulaire allongé creux (1) avec un premier liquide (5) disposé à l'intérieur du manche tubulaire allongé creux (1) et un second liquide absorbé jusque dans ledit embout applicateur (4), **caractérisé en ce que** le premier liquide (5) est séparé de l'extrémité ouverte (3) par une substance visqueuse (6), dans lequel, lors de l'ouverture du manche tubulaire allongé creux (1) par le moyen d'ouverture (7), le premier liquide (5) s'écoule à travers la substance visqueuse (6) et jusque dans ledit embout absorbant (4) pour une application avec le second liquide, la substance visqueuse restant essentiellement dans sa position d'origine.

2. Applicateur à deux liquides selon la revendication 1, dans lequel un second embout applicateur est fixé à ladite extrémité scellée (2) du manche tubulaire allongé creux (1).

3. Applicateur à deux liquides selon la revendication 1, dans lequel l'embout applicateur (4) est réalisé dans un matériau absorbant.

4. Applicateur à deux liquides selon la revendication 1, dans lequel ledit moyen d'ouverture (7) est une pliure.

5. Applicateur à deux liquides selon la revendication 3, dans lequel ledit moyen d'ouverture (7) est une pliure.

6. Applicateur à deux liquides selon la revendication 1, dans lequel ladite substance visqueuse (6) est de la silicone.

7. Applicateur à deux liquides selon la revendication 3, dans lequel ladite substance visqueuse (6) est de la silicone.

8. Applicateur à deux liquides selon la revendication 4, dans lequel ladite substance visqueuse (6) est de la silicone.

9. Applicateur à deux liquides selon la revendication 1, dans lequel ledit embout applicateur (4) est saturé dudit second liquide.

10. Applicateur à deux liquides selon la revendication 1, dans lequel ledit embout applicateur (4) est uniquement partiellement saturé dudit second liquide.

11. Applicateur à deux liquides selon la revendication 1, dans lequel ledit moyen d'ouverture (7) est une section cassante et ladite substance visqueuse (6) est de la silicone.

12. Applicateur à deux liquides selon la revendication 11, dans lequel un second embout applicateur est fixé à ladite extrémité scellée (2) du manche tubulaire allongé creux (1).

13. Applicateur à deux liquides selon la revendication 11, dans lequel ledit embout applicateur (4) est saturé dudit second liquide.

14. Applicateur à deux liquides selon la revendication 11, dans lequel ledit embout applicateur (4) est uniquement partiellement saturé dudit second liquide.
